Europäisches Patentamt

European Patent Office

Office européen des brevets

(19)

(11) **EP 1 305 565 B1**

(12) **EUROPÄISCHE PATENTSCHRIFT**

(45) Veröffentlichungstag und Bekanntmachung des Hinweises auf die Patenterteilung:
**06.04.2005 Patentblatt 2005/14**

(21) Anmeldenummer: **01960135.0**

(22) Anmeldetag: **06.08.2001**

(51) Int Cl.⁷: **G01B 5/25**, A61N 5/10

(86) Internationale Anmeldenummer:
**PCT/DE2001/002924**

(87) Internationale Veröffentlichungsnummer:
**WO 2002/012823 (14.02.2002 Gazette 2002/07)**

(54) **LAGE EINER ROTATIONSACHSE ( PATIENTENTISCH, STRAHLENTHERAPIE ) ÜBER DREHWINKEL UND SEHNE DURCH EINEN VERSCHIEBBAREN MARKER**

DETERMINING THE POSITION OF AN AXIS OF ROTATION (PATIENT POSITIONING TABLE, RADIATION THERAPY) ON THE BASIS OF AN ANGLE OF ROTATION AND A CHORD THROUGH A MOVABLE MARK

DETERMINATION DE LA POSITION D'UN AXE DE ROTATION (NOTAMMENT D'UNE TABLE POUR PATIENT UTILISEE EN RADIOTHERAPIE) SUR LA BASE DE L'ANGLE DE PIVOTEMENT ET D'UNE CORDE AU MOYEN D'UN REPERE DEPLACABLE

(84) Benannte Vertragsstaaten:
**AT BE CH CY DE DK ES FI FR GB GR IE IT LI LU MC NL PT SE TR**

(30) Priorität: **04.08.2000 DE 10038836**

(43) Veröffentlichungstag der Anmeldung:
**02.05.2003 Patentblatt 2003/18**

(73) Patentinhaber: **Deutsches Krebsforschungszentrum Stiftung des öffentlichen Rechts 69120 Heidelberg (DE)**

(72) Erfinder:
• **KARGER, Christian 69221 Dossenheim (DE)**
• **JÄCKEL, Oliver 69221 Dossenheim (DE)**
• **HARTMANN, Günther 69120 Heidelberg (DE)**

(74) Vertreter: **Reuther, Martin Patentanwaltskanzlei Liermann-Castell, Gutenbergstrasse 12 52349 Düren (DE)**

(56) Entgegenhaltungen:
**DE-A- 2 940 633          US-A- 4 123 660
US-A- 5 982 492**

• **BRUCE J. GERBI ET AL.: "Maintaining accuracy in stereotactic radiosurgery" INT. J. RADIATION ONCOLOGY BIOL. PHYS., Bd. 32, Nr. 4, 1995, Seiten 1199-1203, XP000905624 (US)**
• **F. COLOMBO ET AL.: "Entwicklung einer stereotaktischen Vorrichtung der zeiten Generation für die Linearbeschleunigerradiochirurgie" ELECTRO MEDICA, Bd. 59, Nr. 3, 1991, Seiten 98-101, XP000231198 Erlangen (DE)**

**Beschreibung**

Die Erfindung betrifft ein Verfahren und eine Messanordnung zur Bestimmung der Lage einer Rotationsachse eines Körpers bezüglich eines Raumpunktes sowie ein Verfahren zum Ausrichten eines um eine Rotationsachse rotierbaren Patiententisches.

Hierbei betrifft die Erfindung insbesondere das Gebiet der Strahlentherapie. In der Regel weisen entsprechende Bestrahlungseinrichtungen lineare Beschleuniger auf, die auf ein Bestrahlungszentrum gerichtet sind, wobei die vorliegende Erfindung auch für andere Strahlenquellen anwendbar ist.

Bei derartigen Einrichtungen lässt sich ein Isozentrum als Schnittpunkt mehrerer Achsen, beispielsweise einer Drehachse einer Strahlenquellenhalterung oder eines Haltebogens, einer Achse eines Kollimatorkopfes, einer Strahlachse oder einer Rotationsachse eines Patiententisches, definieren. Dieses Isozentrum erweist sich in der Praxis als das Bestrahlungszentrum, also als die Position, in welcher die Bestrahlung während der Behandlung fokussiert wird.

Hierbei ist es insbesondere für die Präzisionsstrahlentherapie, welche nicht mehr ausschließlich Gegenstand der Forschung ist, sondern bereits in die allgemeine Gesundheitsvorsorge Eingang gefunden hat, von Bedeutung, dass diese Achsen sich möglichst genau im Isozentrum treffen, wobei in der Praxis Abweichungen von mehreren Millimetern vorkommen können. Für die Präzisionsstrahlentherapie ist jedoch eine exakte Justierung erforderlich, dieses gilt insbesondere für die Rotationsachse eines Patiententisches bezüglich der übrigen Bestrahlungseinrichtung. Hierbei wird diese Rotationsachse in der Regel so justiert, dass sie vertikal durch das Isozentrum verläuft. Die exakte Justierung ist im allgemeinen äußerst schwierig, da die Tischachse bzw. Rotationsachse selbst nicht sichtbar und insbesondere auch mechanisch nur äußerst schwer kontrollierbar bzw. festlegbar ist.

Zur Justierung der Tischachse sieht beispielsweise die Veröffentlichung von G.H. Hartmann, "Quality assurance program on stereotactic radiosurgery" (Springer-Verlag, Heidelberg, 1995) vor, im Rahmen einer umfassenden Überprüfung der Justierung einer Bestrahlungseinheit eine Testspitze entlang der unsichtbaren Tischachse zu justieren. Dies erfolgt dadurch, dass die Spitze iterativ auf dem Tisch verschoben wird, bis diese bei einer Tischrotation in Bezug auf den Raum nicht mehr auswandert. Anschließend wird die Spitze in das Isozentrum verschoben und die Distanz gemessen. Dieses Verfahren hat jedoch den Nachteil, dass verhältnismäßig zeitaufwendig die Testspitze iterativ positioniert werden muss.

Darüber hinaus muss bei der Überprüfung, ob die Spitze wirklich auf der Tischachse liegt, eine sehr kleine Bewegung der Spitze bei Rotation des Tisches im Bezug auf den umgebenden Raum festgestellt werden, was verhältnismäßig große Anforderungen an die Messgenauigkeit stellt. Auch kann es wegen der großen Masse des Tisches vorkommen, dass die Tischachse bei der Rotation nicht völlig starr im Raum liegt, sodass die Spitze in der Mitte dieser sogenannten Wobbelbewegung positioniert werden muss, um die mittlere Position der Tischachse zu kennzeichnen.

Ein weiteres Verfahren zur Korrektur der Auswanderung eines in Isozentrum positionierten Zielpunktes nach einer Tischrotation wird in der Veröffentlichung von Brezovich I.A., Pareek P.N., Plott W.E. und Jenelle R.L.S., "Quality assurance to correct for Errors arising from couch rotation in linac-based stereotactic radiosurgery" (International Journal of Radiation Oncology Biology Physics 38, 883 bis 890, 1997) beschrieben. Hierbei wird zunächst die Einstellung des Patienten vorher simuliert. Dieses geschieht, indem eine Testspitze unter der Tischstellung von 0° im Isozentrum positioniert und nach der jeweiligen Rotation die Verschiebung zurück ins Isozentrum messen wird. Die auf diese Weise bestimmte Korrektur wird anschließend an Patienten auf dieselbe Weise umgesetzt. Insofern handelt es sich hierbei um eine Simulation der Patientenpositionierung mit anschließender Korrektur, die jedes Mal bei jedem individuellen Patienten durchgeführt werden muss. Hierzu muss regelmäßig explizit jeder Tischwinkel separat vermessen werden, was verhältnismäßig zeitaufwendig ist. Ebenso muss die Korrektur jeder Patientenbewegung durchgeführt werden.

Andererseits ist es aus der DE 29 40 633 A1 bekannt, die Lage einer Rotationsachse eines Körpers zu bestimmen, indem der Körper von einer Ausgangstellung ausgehend in zwei unterschiedliche Winkelstellungen rotiert wird, wobei an dem Körper ein Marker vorgesehen ist und die jeweilige Position des Markers gemessen wird. Aus den drei auf diese Weise ermittelten Punkten kann eine Ebene bestimmt werden, in welcher diese drei Punkte liegen. Hieraus folgt die Richtung der Rotationsachse. Der Schnittpunkt der Rotationsachse mit dieser Ebene folgt, indem durch die drei Punkte ein in dieser Ebene liegender Kreis bestimmt wird, wobei der Mittelpunkt diesen Schnittpunkt definiert. Wegen der Notwendigkeit, drei Punkte zu bestimmen, gestaltet sich dieses Verfahren als verhältnismäßig aufwendig, insbesondere wenn lediglich der Abstand der Rotationsachse von dem Isozentrum benötigt wird.

Es ist Aufgabe vorliegender Erfindung, die Verfahrensabläufe, die bei der Nutzung einer Bestrahlungseinrichtung auftreten, zu vereinfachen bzw. zu beschleunigen.

Als Lösung schlägt die Erfindung einerseits ein Verfahren zur Bestimmung der Lage einer Rotationsachse eines Körpers bezüglich eines Raumpunktes, insbesondere Verfahren zur Bestimmung der Lage einer Rotationsachse eines Patiententisches bezüglich des Isozentrumes, vor, bei welchem der Raumpunkt zunächst mit einem an dem Körper bzw. an dem Patiententisch befindlichen Marker markiert und anschließend eine Rotation des Körpers bzw. des Patiententisches um einem gewählten Winkel durchgerührt und die hierbei von dem Marker zurückgelegte Strecke gemessen wird, wobei anhand der zurückgelegten Strecke und des gewählten Rotationswinkels der Abstandsvektor zwischen dem Raumpunkt und der Rotationsachse errechnet wird.

In der Regel ist bei Bestrahlungseinrichtung der wesentliche Raumpunkt, sprich das Isozentrum, bereits durch Laserlichtstrahlen, die sich im Isozentrum kreuzen, oder ähnliche markiert, wobei häufig die Strahlen zu Ebenen, welche XY-, XZ- und YZ- Ebenen darstellen, aufgefächert sind. Insofern braucht der Marker lediglich in geeigneter Weise an diese Strahlen angepasst werden.

Aus dem Abstandsvektor lässt sich ohne weiteres der Betrag des Abstands sowie die Richtung ermitteln, so dass die Lage der Rotationsachse ohne weiteres bestimmbar ist. Es versteht sich hierbei, dass der Begriff Abstandsvektor in seiner allgemeinsten Bedeutung zu verstehen ist, also als die Angabe über Richtung und Betrag. Andererseits ist es denkbar, dass, wenn nur der Abstand oder nur die Richtung von Interesse ist, nicht unbedingt der Vektor als solches sondern auch unmittelbar die gesuchten Größen errechnet werden können.

Im Gegensatz zu den bereits bekannten Verfahren wird bei dem erfindungsgemäßen Lagebestimmungsverfahren in einem Messschritt die Lage der Rotationsachse bestimmt und steht somit zur weiteren Bearbeitung zur Verfügung. Die bis dato bekannten Verfahren benötigen mehr Messpunkte bzw. gehen einerseits iterativ vor oder speichern lediglich Korrekturwerte, die dann bei jeder Behandlung wieder abgerufen werden müssen.

Dementsprechend schlägt die Erfindung des weiteren ein Verfahren zum Ausrichten eines um eine Rotationsachse rotierbaren Patiententisches, insbesondere für die Strahlentherapie, vor, bei welchem zunächst die Lage der Rotationsachse wie vorbeansprucht bestimmt und anschließend der Tisch derart ausgerichtet wird, dass die Rotationsachse sich in ihrer gewünschten Position befindet.

Bei einer derartigen Verfahrensführung, die insbesondere mittels des vorstehend beschriebenen Lagebestimmungsverfahrens durchgeführt werden kann, ist es möglich, auf eine nachträgliche Korrekturbewegung des Patiententisches bei jedem Handlungsschritt zu verzichten, da die Rotationsachse des Tisches genau positioniert, das heißt im Rahmen der Messgenauigkeit in das Isozentrum gelegt, werden kann. Bei einer Rotation des Tisches verbleibt somit der Patient bezüglich des Bestrahlungszentrums im Rahmen der Messgenauigkeit positioniert, sodass die Behandlungszeit verkürzt und somit die Behandlung für den Patienten angenehmer gestaltet werden können.

Da der betreffende Raumpunkt, insbesondere das Isozentrum, bereits durch den Marker markiert ist und somit geeignete Messeinrichtungen vorliegen, um den Marker in seiner markierenden Funktion verwenden zu können, kann der Marker zur Streckenmessung der zurückgelegte Strecke in den betreffenden Raumpunkt, insbesondere das Isozentrum, zurückbewegt und die hierbei zurückgelegte Rückbewegungsstrecke gemessen werden.

Insofern brauchen bei dieser Verfahrensführung zur erfindungsgemäßen Streckenmessung keine zusätzlichen Messeinrichtungen zur Detektion des Markers vorgesehen sein. Es sind lediglich noch Einrichtungen vorzusehen, welche die zurückgelegte Rückbewegungstrecke messen können. Dieses können beispielsweise Entfernungsmesser, Schrittmotoren oder die Verstell- und Messeinrichtungen eines x-y-Messtisches sein.

Diese Vorgehensweise hat insbesondere den Vorteil, dass genau die Maßnahmen genutzt werden können, welche ohnehin zur Ortsmessung des Markers in dem gewählten Raumpunkt genutzt werden. Insofern braucht nicht ein neuer Raumpunkt durch die entsprechende Messeinrichtung bzw. durch die Maßnahmen zur Ortsmessung des Raumpunktes angefahren und justiert werden. Insofern erleichtert sich hierdurch die Verfahrensführung erheblich.

Vorzugsweise wird zur Lagebestimmung der Abstandsvektor jeweils für verschiedene Rotationswinkel ermittelt, also der Marker von dem Raumpunkt aus durch eine Rotation des Körpers um verschiedene Winkel bewegt, die hierbei von dem Marker zurückgelegte Strecke gemessen und anhand der zurückgelegten Strecke und des jeweiligen Winkels der Abstandsvektor errechnet. Auf diese Weise lässt sich bestimmen, ob bzw. wie weit die Rotationsachse während der Rotation variiert bzw. wobbelt. Eine derartige Variation der Rotationsachse ist insbesondere bei schweren Patiententischen nicht auszuschließen.

Je nach Maß der Variation kann es genügen, aus den ermittelten Werten eine mittlere Lage der Rotationsachse zu bestimmen und diese bzw. den Patiententisch entsprechend auszurichten. Andererseits können diese Ergebnisse Anlass dafür sein, die Mechanik des Patiententisches bzw. des Körpers in geeigneter Weise zu modifizieren, um die Rotationsachse zu stabilisieren. Darüber hinaus ist es auch denkbar, diese durch die Rotation bedingten Abweichungen mittels geeigneter Translationsbewegungen zu kompensieren.

Da jedoch in erfindungsgemäßer Weise die Rotationsachse bereits bestmöglich ausgerichtet ist, sind derartige Kompensationsbewegungen wesentlich kleiner als Kompensationsbewegungen, die bei einer nicht ausgerichteten Rotationsachse auftreten, so dass durch eine Nachkompensation, wie sie bei dem erfindungsgemäßen Verfahren durchgeführt werden kann, die Behandlungszeit nur unwesentlich verlängert wird.

Zur Lagebestimmung kann wenigstens ein zweiter Abstandsvektor zwischen der Rotationsachse und wenigstens einem zweiten Raumpunkt, der um die Haupterstreckungsrichtung der Rotationsachse bezüglich des ersten Raumpunktes versetzt ist, bestimmt und aus den bestimmten Abstandsvektoren und den Raumpunkten die Erstreckungsrichtung der Rotationsachse errechnet werden. Hierbei braucht lediglich durch die Fußpunkte der beiden Abstandsvektoren, die zu den jeweiligen Raumpunkten weisen, eine Gerade gelegt werden.

Werden mehr als zwei Raumpunkte, insbesondere drei Raumpunkte, und eine entsprechende Zahl an Abstandsvektoren ermittelt, so erfolgt die Errechnung der Erstreckungsrichtung der Rotationsachse vorzugsweise durch geeignete statistische Methoden. Es versteht sich, dass auch diese Informationen insbesondere genutzt werden können, um den Körper bzw. den Patententisch in geeigneter Weise auszurichten.

Es versteht sich hierbei, dass die Haupterstreckungsrichtung nicht vorbestimmt werden muss, da dieses gerade durch die beschriebene Lagebestimmung erfolgt. Vielmehr genügt ein grober Versatz des zweiten Raumpunktes in diese Richtung, da die genaue Messung dann durch die Ermittlung des Abstandsvektors erfolgt. Es versteht sich hierbei, das jedoch die Lage des derartig versetzen Raumpunktes ausreichend genau bestimmt sein muss.

Vorzugsweise wird die Lage der Rotationsachse in gewählten Zeitabständen bestimmt und der Patententisch gegebenenfalls nachgerichtet. Durch eine derartige Vorgehensweise ist es möglich, dass Veränderungen nachkorrigiert werden, ohne dass vor jeder Behandlung eine Kalibrierung der Bestrahlungseinrichtung vorgenommen werden muss, wie diese beim Stand der Technik vorgesehen ist.

Als weitere Lösung schlägt die Erfindung eine Messanordnung zur Bestimmung der Lage einer Rotationsachse eines Körpers bezüglich eines Raumpunktes, insbesondere eines Patententisches bezüglich eines Isozentrums, mit einem Messtisch vor, der über Positionierungsmittel bezüglich des Körpers positioniert ist und der eine Messspitze, eine Einrichtung zum Verstellen der Messspitze bezüglich der Positionierungsmittel in zumindest zwei Dimensionen sowie eine Einrichtung zum Bestimmen der verstellten Strecke umfasst.

Mit einer derartigen Anordnung kann ohne weiteres das Lagebestimmungsverfahren, welches vorstehend beschrieben worden ist, durchgeführt werden. Insbesondere kann mit einer derartigen Anordnung dieses Verfahren verhältnismäßig einfach und schnell durchgeführt werden.

Der Begriff "Dimensionen" erstreckt sich in vorliegendem Zusammenhang auf sämtliche Raumdimensionen, seien es kartesische Koordinaten, Zylinderkoordinaten oder Kugelkoordinaten, erstrecken kann, wobei diese nicht zwingend ortsfest vorgesehen sein müssen. Wesentlich jedoch ist, dass die Messspitze bezüglich der Positionierungsmittel in zwei linear unabhängigen Dimensionen verstellbar sein soll.

Auch versteht es sich, dass auch andere Koordinatensysteme Verwendung finden können, solange diese durch geeignete Transformationen in die Koordinatensysteme dieser Erfindungsbeschreibung zurückgeführt werden können.

Die Einrichtung zum Bestimmen der verstellten Strecke kann jede übliche Messanordnung zur Streckenbestimmung, wie geeignet Entfernungsmesser, Verstell- oder Schrittmotoren oder ähnliches, umfassen, solange lediglich die beim Verstellen zurückgelegten Strecke mit ausreichender Messgenauigkeit zugänglich ist.

Weist der Messtisch eine Verstelleinrichtung zum Verstellen der Messspitze bezüglich der Positionierungsmittel in zumindest drei Dimensionen auf, so kann mit dieser Messanordnung ohne weiteres auch die Erstreckungsrichtung der Rotationsachse ermittelt werden, wie dieses bereits vorstehend beschrieben ist.

Wird die erfindungsgemäße Messanordnung im Zusammenspiel mit einem in wesentlichen horizontal angeordneten Tisch, insbesondere einem Patententisch, genutzt, der um eine Rotationsachse, die eine vertikale Komponente aufweist, rotierbar ist, so können die Positionierungsmittel eine Auflage umfassen, mittels welcher der Messtisch auf dem Patententisch aufliegt bzw. auf diesen aufgelegt wird. Durch die horizontale Ausrichtung des Tisches bleibt der Messtisch in seiner Position auf dem Patententisch, wenn dieser rotiert. Es versteht sich, das eine derartige Anordnung außerordentlich einfach und somit kostengünstig baut.

Durch geeignete Wahl des Messtisches bzw. der Verstelleinrichtung und der Einrichtung zum Bestimmen der verstellten Strecke, für welche im übrigen beispielsweise ein bekannter x-y-Messtisch verwendet werden kann, kann die Messanordnung darüber hinaus äußerst kostengünstig realisiert werden.

Die Messanordnung baut darüber hinaus verhältnismäßig einfach und kostengünstig, wenn sich kreuzende Strahlen als Mittel zum Markieren des Raumpunktes genutzt werden. Derartige, sich kreuzende Strahlen können vorzugsweise Laserlichtstrahlen sein, die ohne weiteres mit großer Genauigkeit ausgerichtet werden können.

Darüber hinaus weisen Bestrahlungseinrichtungen häufig Laserlichtstrahlen zur Markierung des Isozentrums auf, so dass diese Laserlichtstrahlen in entsprechende Weise genutzt werden können. Um eine einfache Justierung der Messspitze zu gewährleisten, kann diese einen Messkopf aufweisen, welcher mit diesen Strahlen in geeigneter Weise wechselwirkt. Hierbei kann der Messkopf beispielsweise derart gewählt werden, dass er die Strahlen sichtbar macht, wodurch der Messkopf bzw. die Messspitze verhältnismäßig einfach in dem Kreuzungspunkt justiert werden.

Weiter Vorteile, Ziele und Eigenschaften der vorliegenden Erfindung werden anhand der Beschreibung anliegender Zeichnung erläutert.

In der Zeichnung zeigt:

Figur 1    eine perspektivische Schemadarstellung eines Messtisches für eine erfindungsgemäße Messanordnung und

Figur 2    eine Darstellung der geometrischen Verhältnisse während der Lagebestimmung.

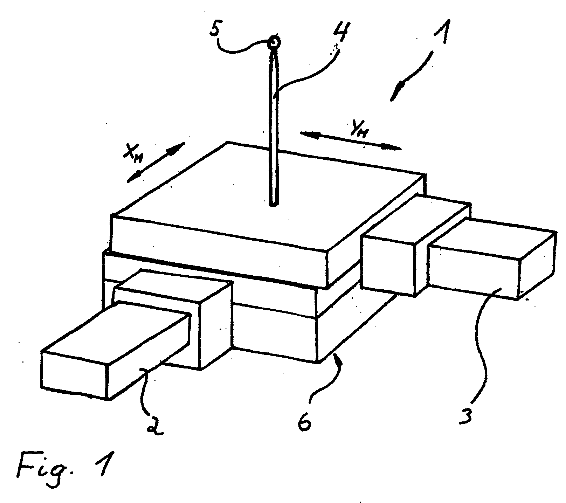

Bei dem in Figur 1 dargestellten Messtisch 1 handelt es sich um einen x-y-Messtisch, an welchem über zwei Verstelleinrichtungen 2, 3 eine Messspitze 4 in zwei Dimensionen, nämlich $X_M$ und $Y_M$, verstellt werden kann. Die Verstelleinrichtungen 2, 3 sind Mikrometertriebe, welche mit einer Genauigkeit von 0,01 mm die Messspitze positionieren können und in der Lage sind, ihre Verstellposition bzw. die von ihre durchlaufene Strecke auszugeben.

An der Messspitze ist eine Kugel 5 mit einem Durchmesser von 5 mm als Messkopf befestigt, welche mit einer orangenen Farbe bedeckt ist, welche mit Laserlichtlinien einer Bestrahlungseinrichtung derart besonders wechselwirkt, dass diese Laserlichtlinien optimal sichtbar sind. Es versteht sich, dass bei anderen Ausführungsformen je nach verwandten Strahlen bzw. Laserstrahlen andere Maßnahmen bzw. andere Farben zur Darstellung eines Markers verwandt werden können.

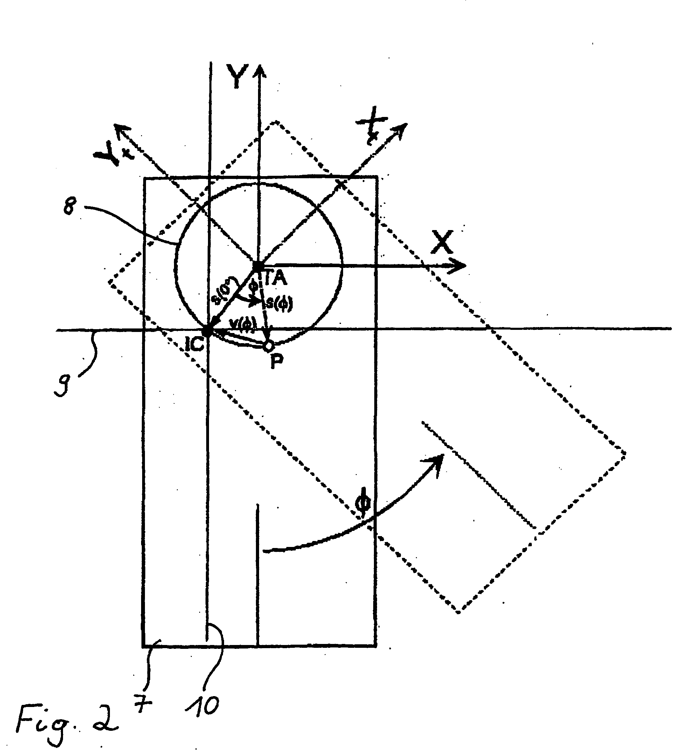

Der Messtisch 1 weist eine plane Unterseite 6 auf, die als Auflage dient, um den Messtisch 1 auf einem Patiententisch 7 (siehe Figur 2) zu positionieren.

Vorzugsweise wird der Messtisch 1, wenn er auf den Patiententisch 7 aufgesetzt wird, derart ausgerichtet, dass die Achsen $X_M$ und $Y_M$ entlang der Tischachsen $X_t$ und $Y_t$ ausgerichtet sind. Auf diese Weise kann auf eine weitere Korrektur bei einer nachfolgend durchzuführenden Koordinatentransformation verzichtet werden.

In einer bevorzugten Ausführungsform umfassen die erfindungsgemäßen Positionierungsmittel Ausrichtmittel, wie geeignet Nut-Feder-Anordnungen, Ausnehmungen und/oder Stift-Loch-Steckverbindungen, die ein derartiges Ausrichten erleichtern bzw. nicht erforderlich machen.

Nachdem der Messtisch 1 auf dem Patiententisch 7 positioniert ist, wird der Messkopf 5 in das Isozentrum der Bestrahlungseinrichtung gebracht. Dieses kann einerseits durch Verlagern des gesamten Messtisches 1 erfolgen. Andererseits können hierfür auch die Verstelleinrichtungen 2 und 3 des Messtisches 1 genutzt werden.

Ist der Messkopf 5 in dem Isozentrum der Bestrahlungseinrichtung angeordnet, so ist er um den Abstandsvektor s(0) von der Achse TA (table axis) beabstandet. Genau diesen Abstand bzw. Abstandsvektor gilt es zu ermitteln. Hierbei wird dieser Abstandsvektor s in einem raumfesten Koordinatensystem X, Y, Z definiert und dreht in einem zu dem Patiententisch 7 ortsfesten Koordinatensystem $X_t$, $Y_t$, $Z_t$ mit. Wird nunmehr der Patiententisch 7 um einen Winkel $\phi$ um die Rotationsachse TA gedreht, so folgt der Messkopf 5 dieser Rotation entlang einer Kreisbahn 8. Nach Durchlaufen des Drehwinkels $\phi$ gelangt der Messkopf 5 somit zu dem Punkt P, welcher im raumfesten Koordinatensystem X, Y, Z durch den Abstandsvektor $s(\phi)$ bezeichnet ist. Hierbei folgt $s(\phi)$ durch

$$s(\phi) = R(\phi)s(0°)$$

unter der Rotationsmatrix $R(\phi)$

$$R(\phi) = \begin{pmatrix} \cos\phi & -\sin\phi \\ \sin\phi & \cos\phi \end{pmatrix}$$

in Abhängigkeit von dem Rotationswinkel $\phi$. Wie unmittelbar aus Figur 2 ersichtlich, bezeichnet der Vektor $v(\phi)$, für den die folgende Bedingung gilt

$$s(0°) = s(\phi) + v(\phi)$$

und welcher von dem Punkt P zum Isozentrum IC reicht, die Strecke, welche benötigt wird, um den Messkopf 5 wieder in das Isozentrum IC zu verlagern. Bei vorliegender Messanordnung wird diese Rückbewegungsstrecke $v(\phi)$ durch Verstellen der Verstelleinrichtungen 2 und 3 durchlaufen, wobei die durchlaufende Strecke in Koordinaten des Patiententisches $X_t$, $Y_t$, $Z_t$ angegeben ist und dementsprechend mit $v_t(\phi)$ bezeichnet ist. Hierbei folgt durch Koordinatentransformation, dass

$$v(\phi) = R(\phi)v_t(\phi)$$

ist. Eine entsprechende Rücktransformation ergibt

$$s(0°) = (R(-\phi)-1)^{-1}v_t(\phi)$$

bzw.

$$s_X(0°) = -\frac{1}{2}\left(v_{t,x}(\phi) + v_{t,Y}(\phi)\cot\frac{\phi}{2}\right)$$

$$s_Y(0°) = \frac{1}{2}\left(v_{t,x}(\phi)\cot\frac{\phi}{2} - v_{t,Y}(\phi)\right)$$

in einzelnen Koordinaten für den Abstandsvektor $s(0°)$ von der Rotationsachse TA des Patiententisches zum Isozentrum.

Es versteht sich, dass durch Wahl verschiede-

ner Rotationswinkel φ ein Mittelwert errechnet bzw. eine Verlagerung der Translationsachse in Abhängigkeit von einem Rotationswinkel φ bestimmt werden kann. Durch diese Angaben können - bei größeren Abweichungen - geeignete Maßnahmen, wie eine verbesserte Lagerung oder ähnliches, vorgesehen werden, um derartige Abweichungen zu vermeiden. Sind die Abweichungen innerhalb der gewünschten Genauigkeit, so können diese toleriert und der Mittelwert zur Ausrichtung genutzt werden.

Durch Verändern der Höhe der Messspitze 4 kann die Erstreckungsrichtung der Rotationsachse TA ermittelt werden. Hierbei wird der Messkopf 5 nicht im Isozentrum IC, sondern im Schnittpunkt der X-Isolinie 9 und der Y-Isolinie 10, die durch Laserlichtstrahlen bezeichnet werden, angeordnet, also in Z-Richtung bzw. der Erstreckungsrichtung der Rotationsachse TA gegenüber dem Isozentrum IC versetzt. Auch hier kann durch geeignete Wahl der Messpunktezahl ein Mittelwert für die Erstreckungsrichtung sowie eine entsprechende statistische Abweichung ermittelt werden, die Aussagen über die Genauigkeit machen.

## Patentansprüche

1. Verfahren zur Bestimmung der Lage einer Rotationsachse (TA) eines Körpers (7) bezüglich eines Raumpunktes (IC), bei welchem der Raumpunkt (IC) zunächst mit einem an dem Körper (7) befindlichen Marker (5) markiert und anschließend eine Rotation des Körpers (7) um einen gewählten Winkel (φ) durchgeführt und die hierbei von dem Marker (5) zurückgelegte Strecke ($v_t$) gemessen wird, *dadurch gekennzeichnet, dass* anhand der zurückgelegten Strecke ($v_t$) und des gewählten Rotationswinkels (φ) der Abstandsvektor (s(0)) zwischen dem Raumpunkt (IC) und der Rotationsachse (TA) errechnet wird.

2. Lagebestimmungsverfahren nach Anspruch 1, *dadurch gekennzeichnet, dass* zur einer Messung der zurückgelegten Strecke ($v_t$) der Marker (5) in den Raumpunkt (IC) zurückbewegt und die hierbei zurückgelegte Rückbewegungsstrecke gemessen wird.

3. Lagebestimmungsverfahren nach Anspruch 1 oder 2, *dadurch ge kennzeichnet, dass* zur Lagebestimmung der Abstandsvektor für verschiedene Rotationswinkel ermittelt wird.

4. Lagebestimmungsverfahren nach einem der Ansprüche 1 bis 3, *dadurch gekennzeichnet, dass* zur Lagebestimmung wenigstens ein zweiter Abstandvektor zwischen der Rotationsachse (TA) und wenigstens einem zweiten Raumpunkt, der um die Haupterstreckungsrichtung der Rotationsachse (TA) bezüglich des ersten Raumpunktes (IC) versetzt ist, bestimmt und aus den Abstandsvektoren und den Raumpunkten die Erstreckungsrichtung der Rotationsachse errechnet wird.

5. Verfahren zum Ausrichten eines um eine Rotationsachse (TA) rotierbaren Patiententisches, insbesondere für die Strahlentherapie, bei welchem zunächst die Lage der Rotationsachse (TA) mittels eines Lagebestimmungsverfahrens nach einem der Ansprüche 1 bis 4 bestimmt und anschließend der Tisch (7) derart ausgerichtet wird, dass die Rotationsachse (TA) sich in ihrer gewünschten Position befindet.

6. Verfahren nach Anspruch 5, *dadurch gekennzeichnet, dass* die Lage der Rotationsachse (TA) in gewählten Zeitabständen bestimmt und der Tisch (7) gegebenenfalls nachgerichtet wird.

7. Messanordnung zur Bestimmung der Lage einer Rotationsachse (TA) eines Körpers (7) bezüglich eines Raumpunktes (IC), *gekenn-zeichnet durch* einen Messtisch (1), der über Positionierungsmittel (6) bezüglich des Körpers (7) positioniert ist und der eine Messspitze (4), einer Einrichtung (2, 3) zum Verstellen der Messspitze bezüglich der Positionierungsmittel (6) in zumindest zwei Dimensionen ($X_M$, $Y_M$) sowie eine Einrichtung zum Bestimmen der verstellten Strecke ($v_t$) umfasst.

8. Messanordnung nach Anspruch 7, *gekennzeichnet durch* eine Verstelleinrichtung zum Verstellen der Messspitze (4) bezüglich der Positionierungsmittel (6) in zumindest drei Dimensionen ($X_M$, $Y_M$, Z).

9. Messanordnung nach Anspruch 7 oder 8, *dadurch gekennzeichnet, dass* der Körper (7) einen im wesentlichen horizontal angeordneten Tisch (7) umfasst, der um eine Rotationsachse (TA), die eine vertikale Komponente aufweist, rotierbar ist, und dass die Positionierungsmittel (6) eine Auflage (6) umfassen, mittels welcher der Messtisch (1) auf dem Tisch (7) aufliegt.

10. Messanordnung nach einem der Ansprüche 7 oder 9, *gekennzeichnet durch* Mittel zum Markieren des Raumpunktes mittels sich kreuzender Strahlen, vorzugsweise Laserlichtstrahlen, und **durch** einen mit diesen Strahlen wechselwirkenden, und diese vorzugsweise sichtbar machenden, an der Messspitze angeordneten Messkopf (5).

## Claims

1. Method for determining the position of an axis of rotation (TA) of a body (7) with reference to a point in space (IC), in which the point in space (IC) first marks with a marker (5) located on the body (7) and next performs a rotation of the body (7) about a selected angle (Φ) and the covered distance ($v_t$) determined in this manner is measured, **characterized in that** with reference to the covered distance ($v_t$) and the selected angle of rotation (Φ), the distance vector (s(0)) between the point in space (IC) and the axis of rotation (TA) is calculated.

2. Method for determining position according to claim 1, **characterized in that** for a measurement of the covered distance ($v_t$), the marker (5) moves back into the point in space (IC) and the distance of the return movement is measured.

3. Method for determining position according to claim 1 or 2, **characterized in that** for determining position, the distance vector for several angles of rotation is measured.

4. Method for determining position according to one of claims 1 through 3, **characterized in that** for determining position, at least one second distance vector between the axis of rotation (TA) and at least one second point in space, which is offset about the primary extension direction of the axis of rotation (TA) with reference to the first point in space (IC), is determined and from the distance vectors and the points in space, the extension direction of the axis of rotation is calculated.

5. Method for adjustment of a patient table that is rotatable about an axis of rotation (TA), in particular, for radiation therapy, in which first the position of the axis of rotation (TA) is determined by means of a method for determining position according to one of claims 1 through 4 and next the table (7) is adjusted, such that the axis of rotation (TA) is located in its desired position.

6. Method according to claim 5, **characterized in that** the position of the axis of rotation (TA) is determined in a selected time intervals and the table (7) is readjusted if necessary.

7. Measuring assembly for determining the position of an axis of rotation (TA) of a body (7) with reference to a point in space (IC), **characterized in that** a measuring table (1), which is positioned via a positioning means (6) with reference to the body (7) and which includes a measuring point (4), a device (2, 3) for adjusting the measuring point with reference to the positioning means (6) in at least two dimensions ($X_M$, $Y_M$) as well as a device for determining the adjusted distance ($v_t$).

8. Measuring assembly according to claim 7, **characterized by** an adjustment device for adjusting the measuring point (4) with reference to the positioning means (6) in at least three dimensions ($X_M$, $Y_M$, Z).

9. Measuring assembly according to claim 7 or 8, **characterized in that** the body (7) includes an essentially horizontally arranged table (7), which is rotatable about an axis of rotation (TA), which has a vertical component, and that the positioning means (6) includes a support (6), by means of which the measuring table (1) rests on the table (7).

10. Measuring assembly according to one of claims 7 or 9, **characterized by** means for marking the point in space by means of intersecting rays, preferably, laser rays, and by a measuring head arranged on the measuring point that interacts with these rays and which makes these rays visible.

**Revendications**

1. Procédé permettant de déterminer la position d'un axe de rotation (TA) d'un corps (7) par rapport à un point dans l'espace (IC), dans lequel on marque d'abord le point dans l'espace (IC) à l'aide d'un marqueur situé sur le corps (7) et on procède ensuite à une rotation du corps (7) autour d'un angle choisi (φ) et on mesure le trajet($v_t$) parcouru à cet effet par le marqueur (5), **caractérisé en ce qu'**à l'aide du trajet parcouru ($v_t$) et de l'angle de rotation choisi (φ), on calcule le vecteur de distance (s(0)) entre le point dans l'espace (IC) et l'axe de rotation (TA).

2. Procédé de détermination de la position selon la revendication 1, **caractérisé en ce que** pour mesurer le trajet parcouru ($v_t$), on redéplace le marqueur (5) sur le point dans l'espace (IC) et on mesure le trajet de retour parcouru à cet effet.

3. Procédé de détermination de la position selon la revendication 1 ou 2, **caractérisé en ce que** pour déterminer la position, on recherche le vecteur de distance pour différents angles de rotation.

4. Procédé de détermination de la position selon l'une quelconque des revendications 1 à 3, **caractérisé en ce que** pour déterminer la position, on détermine au moins un second vecteur de distance entre l'axe de rotation (TA) et au moins un second point dans l'espace, qui est déporté par rapport au premier point dans l'espace (IC) autour de la direction principale d'extension de l'axe de rotation et à partir des vecteurs de distance et des points dans l'espace, on calcule la direction d'extension de l'axe de rotation.

**5.** Procédé pour l'orientation d'une table pour patient, notamment destinée à la radiothérapie autour d'un axe de rotation (TA), dans lequel on mesure tout d'abord de préférence la position de l'axe de rotation (TA), à l'aide d'un procédé de détermination de la position selon l'une des revendications 1 à 4 et on oriente ensuite la table (7) de façon à ce que l'axe de rotation (TA) se trouve dans sa position souhaitée.

**6.** Procédé selon la revendication 5, **caractérisé en ce qu'**on détermine la position de l'axe de rotation (TA) à des intervalles de temps choisis et on réajuste la table (7) le cas échéant.

**7.** Agencement de mesure pour la détermination de la position d'un axe de rotation (TA) d'un corps (7) par rapport à un point dans l'espace (IC), **caractérisé par** une table de mesure (1) qui est positionnée au dessus de moyens de position (6) par rapport au corps (7) et qui comprend une pointe de mesure (4), un dispositif (2, 3) pour l'ajustage de la pointe de mesure par rapport aux moyens de positionnement (6) dans au moins deux dimensions ($X_M$, $Y_M$), ainsi qu'un dispositif pour déterminer le trajet d'ajustage ($v_t$).

**8.** Agencement de mesure selon la revendication 7, **caractérisé par** un dispositif d'ajustage permettant d'ajuster la pointe de mesure (4) par rapport aux moyens de positionnement (6), dans au moins trois dimensions ($X_M$, $Y_M$, Z).

**9.** Agencement de mesure selon la revendication 7 ou 8, **caractérisé en ce que** le corps (7) comprend une table (7) disposée sensiblement à l'horizontale qui est rotative autour d'un axe de rotation (TA) qui présente une composante verticale et **en ce que** les moyens de positionnement (6) comprennent un support (6), par l'intermédiaire duquel la table de mesure (1) repose sur la table (7).

**10.** Agencement de mesure selon l'une quelconque des revendication 7 ou 9, **caractérisé par** des moyens pour marquer le point dans l'espace à l'aide de rayons qui se croisent, de préférence des rayons de lumière laser et par une tête de mesure (5) disposée sur la pointe de mesure et qui est en interaction avec lesdits rayons et les rend visibles de préférence (5).

Fig. 1

Fig. 2